# EUROPÄISCHE PATENTANMELDUNG

(11) **EP 4 014 962 A1**
(43) Veröffentlichungstag der Anmeldung: **22.06.2022**
(21) Anmeldenummer: 20214780.7
(22) Anmeldetag: 16.12.2020
(51) Int. Cl.: A61K 9/00, A61K 47/02, A61K 47/38, A61K 47/10

(54) **ZUSAMMENSETZUNG MIT OSMOTISCH WIRKSAMER SUBSTANZ UND VERWENDUNG DER ZUSAMMENSETZUNG**

(71) Anmelder: Novigo GmbH & Co. KG, 35390 Gießen (DE); Sidroga AG, 4310 Rheinfelden (CH)
(72) Erfinder: Schmidts, Thomas, 35396 Gießen (DE)
(74) Vertreter: Patentanwälte Olbricht Buchhold Keulertz

(57) **Zusammenfassung**

Die Erfindung betrifft eine Zusammensetzung zur Anwendung bei der Behandlung von Hautreizungen, welche durch pflanzliche oder tierische Einwirkungen auf oder in der Haut verursacht werden, wobei die Zusammensetzung wenigstens eine osmotisch und/oder hygroskopisch wirksame Substanz enthält.

## Beschreibung

Die Erfindung betrifft eine Zusammensetzung gemäß Anspruch 1 sowie eine Verwendung der Zusammensetzung gemäß Anspruch 8.

Hautreizungen hervorgerufen durch tierische oder pflanzliche Einwirkungen können aktuell zumeist nur wenig befriedigend behandelt werden. Zu den tierischen Einwirkungen gehören beispielweise Stiche und Bisse durch Gliederfüßler, insbesondere Insekten und Spinnentiere, sowie die Applikation von Nesselgiften, beispielweise von Raupen der Prozessionspinnen, insbesondere Raupen des Eichenprozessionsspinners sowie Nesseltieren, insbesondere Quallen. Die Applikation von Nesselgiften auf oder in die Haut kann alternativ auch durch die Einwirkung von Brennnesseln erfolgen und somit pflanzlichen Ursprungs sein. Weiterhin können Pflanzen durch Hautkontakt mit Dornen, Stacheln, Nadeln, Haaren und anderen Formen von Emergenzen und Trichomen zu Verletzungen und Reizungen der Haut führen, wie beispielweise Herkulesstauden, Koniferen und Tannen.

Zu den Hautreizungen gehören beispielweise Rötungen, Schwellungen, Quaddeln und Juckreiz im Bereich der von der Einwirkung betroffenen Stelle.

Zu den Insektenbissen und -stichen sind insbesondere Bisse oder Stiche durch Hautflügler (Hymenotera), Zweiflügler (Diptera) und Neuflügler (Neoptera) zu zählen. Die Hautflügler (Hymenotera) umfassen beispielweise Bienen, Wespen und Ameisen. Zu den Zweiflüglern gehören beispielweise Mücken und Bremsen. Die Neuflügler umfassen beispielweise Flöhe, Läuse und Wanzen.

Zu den Spinnentieren (Arachnida) werden neben Spinnen auch Milben und Zecken gezählt.

Zurzeit gibt es einerseits Präparate zur Behandlung der Hautreizung, welche die Symptome, insbesondere den Juckreiz lindern, aber nicht zur Beseitigung der Ursache beitragen. Hierzu gehören beispielweise Präparate, die lediglich durch Kühlung oder durch die Behandlung mit pharmakologisch wirksamen Substanzen, wie beispielweise Cortisonderivate oder Antihistaminika, den Juckreiz lindern. Diese Präparate beseitigen allerdings nicht die Ursache der Hautreizung, insbesondere das von Tieren oder Pflanzen abgesonderte Sekret oder Nesselgift pflanzlichen oder tierischen Ursprungs.

Anderseits gibt es Behandlungsmethoden, die zur Beseitigung der Ursache der Hautreizung beitragen, allerdings aufwendig und unangenehm bis hin zu schmerzhaft sind. Zu diesen Behandlungsmethoden gehören beispielweise die Behandlung einer Einstichstelle mit Hitze oder Unterdruck. Durch das Erhitzen der Einstichstelle soll das Sekret, insbesondere das von Mücken abgesonderte Sekret in Form von Irritantien und/oder Gift, denaturiert werden, wodurch ein Juckreiz verhindert werden soll. Allerdings ist diese Behandlung zumindest kurzzeitig schmerzhaft. Bei der Behandlung der Hautreizung mit Unterdruck, beispielweise durch die Verwendung von Unterdruckpumpen, soll das Sekret durch den Einstichkanal oder Bissstelle entfernt werden. Dies ist allerdings aufwendig und fehleranfällig in der Handhabung und führt zu einer Schädigung der Haut durch den Unterdruck.

Aufgabe der Erfindung ist es daher, die Nachteile des Standes der Technik zu beseitigen und eine topisch anwendbare Zusammensetzung bereitzustellen, die Hautreizungen, hervorgerufen durch pflanzliche und tierische Einwirkungen, insbesondere durch Stiche und Bisse von Insekten und Spinnen sowie durch Nesselgifte, lindert und die Ursache der Hautreizung reduziert bis hin zu entfernt.

Hauptmerkmale der Erfindung sind im kennzeichnenden Teil von Anspruch 1 und 8 angegeben. Ausgestaltungen sind Gegenstand der Ansprüche 2 bis 7 und 9.

Die Erfindung betrifft eine Zusammensetzung zur Behandlung von Hautreizungen, welche durch pflanzliche oder tierische Einwirkungen auf oder in der Haut verursacht werden, wobei die Zusammensetzung wenigstens eine osmotisch und/oder hygroskopisch wirksame Substanz enthält.

Pflanzliche und tierische Einwirkungen auf oder in der Haut umfassen insbesondere die Applikation von Sekret durch Gliederfüßler, beispielweise durch Insektenstiche oder Spinnenbisse, sowie von Nesselgift, beispielweise aus pflanzlichen Quellen oder tierischen Ursprungs, insbesondere von Brennnesseln, Quallen oder Raupen der Prozessionsspinnen. Die Applikation erfolgt dabei beispielweise durch die Verletzung der Haut durch Nesselhaare oder durch Einstich- oder Bissstellen, wobei der die jeweilige Hautreizung auslösende Fremdstoff, insbesondere Sekret oder Gift, zumindest teilweise in die Haut eindringt.

Sekret wird in diesem Dokument für jeden zumindest teilweise flüssigen oder gelösten Fremdstoff tierischen oder pflanzlichen Ursprungs verwendet, beispielweise Speichel, Gifte oder anderweitige Reizstoffe. Sekrete, die beispielweise bei einem Insektenstich in die Haut appliziert werden, wirken irritierend, hautreizend und sind potentiell entzündungsauslösend.

Osmotisch wirksame Substanzen sind Stoffe, für die eine für das Lösungsmittel permeable Membran impermeabel ist. Ein Konzentrationsunterschied zwischen zwei durch eine Membran getrennte Kompartiments, aufgrund von osmotisch wirksamen Substanzen, kann somit nur durch einen Flüssigkeitsfluss ausgeglichen werden.

Hygroskopisch wirksame Substanzen ziehen Feuchtigkeit aus der Umgebung an und haben somit eine ähnliche Sogwirkung auf Flüssigkeiten wie osmotisch wirksame Substanzen.

Dieser Effekt wird zur Behandlung von durch tierische und pflanzliche Einwirkungen verursachte Hautreizungen genutzt. Der von Tieren oder Pflanzen in die Haut applizierte Fremdstoff, welcher die Hautreizung verursacht und nicht einfach mechanisch, beispielweise durch Abwischen, Abspülen der Haut oder Herausziehen eines Stachels entfernt werden kann, wird mittels der osmotisch und/oder hygroskopisch wirksamen Substanz in der topisch angewendeten Zusammensetzung aus der Haut entfernt. Insbesondere wird der in die Haut eingebrachte Fremdstoff, welcher beispielweise ein Sekret oder Nesselgift sein kann, aus der Einstich- oder Bissstelle, gezogen und somit entfernt. Hierdurch wird die Ursache der Hautreizung schmerzfrei und ohne Beschädigung der Haut behandelt.

Die topische Anwendung einer Zusammensetzung zur Behandlung von Hautreizungen durch tierische oder pflanzliche Einwirkungen mit einer osmotisch und/oder hygroskopisch wirksamen Substanz ermöglicht somit die schonende Entfernung eines die Hautreizung verursachenden Fremdstoffes aus der Haut basierend auf dem Konzentrationsunterschied zwischen dem Fremdstoff und der Zusammensetzung. Die Zusammensetzung weist dabei vorzugsweise eine Konzentration an osmotisch und/oder hygroskopisch wirksamer Substanz auf, die höher ist als die Konzentration in dem Fremdstoff, insbesondere Sekret oder Gift, oder der physiologischen Konzentration der Haut.

Eine Zusammensetzung ist dabei eine Kombination von mehreren Substanzen und Inhaltsstoffen, die zusammen ein auf Haut, insbesondere im Bereich einer Hautreizung, einer Stich- oder Bissstelle, zur topischen Behandlung aufbringbares Präparat bilden.

Die Haut, die eine Hautreizung aufweist ist insbesondere menschlichen und/oder tierischen Ursprunges. Somit ist die Zusammensetzung zur Behandlung von Hautreizungen, insbesondere verursacht durch Stiche oder Bisse von Gliederfüßlern oder auch pflanzliche oder tierische Nesselgifte am Menschen und/oder Tier geeignet.

Vorzugsweise ist wenigstens eine osmotisch und/oder hygroskopisch wirksame Substanz in der Zusammensetzung ein Salz oder eine niedermolekulare organische Substanz, beispielweise ein Zucker.

Hierdurch kann eine Zusammensetzung zur Applikation auf die Haut im Bereich einer Hautreizung erhalten werden, welche die Ursachen der Hautreizung reduziert bis hin zu vollständig entfernt und zugleich für die Haut gut verträglich ist, ohne negative Nebenwirkungen aufzuweisen.

In einer bevorzugten Ausführungsform enthält die Zusammensetzung mehrere osmotisch und/oder hygroskopisch wirksame Substanzen. Sind mehrere osmotisch und/oder hygroskopisch wirksame Substanzen in der Zusammensetzung enthalten, können beispielweise - zwei oder mehr verschiedene Salze - oder - zwei oder mehrere verschiedene Zucker - oder - ein oder mehrere Salze in Kombination mit einem oder mehreren Zuckern - enthalten sein. Durch die Verwendung mehrerer Salze oder Zucker in der Zusammensetzung können neben der osmotischen und/oder hygroskopischen Wirksamkeit weitere vorteilhafte Eigenschaften kumuliert ausgenutzt werden. So können beispielweise neben osmotisch und/oder hygroskopisch hochwirksamen Salzen zusätzlich Salze eingesetzt werden, die eine zusätzliche entzündungshemmende Wirkung haben.

In einer bevorzugten Ausführungsform umfasst oder besteht die osmotisch und/oder hygroskopisch wirksame Substanz ein nicht giftiges anorganisches oder organisches Salz. Insbesondere weist ein anorganisches Salz als osmotisch und/oder hygroskopisch Substanz ein Kation ausgewählt aus der Gruppe: Natrium, Kalium, Lithium, Magnesium, und/oder Calcium und ein Anion ausgewählt aus der Gruppe Sulfat, Sulfid, Chlorid, Bromid, lodid, Fluorid oder Carbonat auf. Ein organisches Salz osmotisch und/oder hygroskopisch Substanz weist insbesondere ein Kation ausgewählt aus der Gruppe der quartärer Ammoniumverbindungen oder organisch substituierten Ammoniumverbindungen und vorzugsweise ein Anion ausgewählt aus der Gruppe der Carboxylate oder organischer Sulfate auf. Alternativ umfasst oder besteht die osmotisch und/oder hygroskopisch wirksame Substanz ein Salz aus der Gruppe der Aminosäuren oder Betaine.

In einer alternativen oder ergänzenden Ausführungsform umfasst oder besteht die osmotisch und/oder hygroskopisch wirksame Substanz einen Zucker, insbesondere ein oder mehrere Mono- und/oder Disaccharide, Zuckeralkohole, insbesondere Mannit, Sorbit, Xylit und/oder Isomalt.

Besonders bevorzugt enthält die Zusammensetzung ein Mineralsalz als osmotisch und hygroskopisch wirksame Substanz. Mineralsalz bezeichnet eine aus mehreren anorganischen Verbindungen und Salzen bestehende Zusammensetzung. Hierzu gehört beispielweise Mineralsalz aus Thermalquellen der Stadt Bad Ems, welches auch unter Bezeichnung Emser Salz ^{®} im Handel erhältlich ist. Emser Salz ^{®} weist neben Natriumchlorid und Hydrogencarbonat mehr als 30 Mineralstoffe und Spurenelemente auf.

Emser Salz als Mineralsalz umfasst pro kg Mineralsalz gemäß Analyse 308,7 g Natrium-Ionen, 6,11 g Kalium-Ionen, 0,291 g Magnesium-Ionen, 0,21 g Lithium-Ionen, 0,016 g Calcium-Ionen, 0,003 g Eisen(II, III)-Ionen, 0,0001 g Mangan-Ionen, 474,4 g Hydrogencarbonat-Ionen, 188,4 g Chlorid-Ionen, 14,0 g Carbonat-Ionen, 9,24 g Sulfat-Ionen, 0,355 g Nitrat-Ionen, 0,202 g Bromid-Ionen, 0,078 g Fluorid-Ionen, 0,005 g lodid-lonen.

Vorzugsweise enthält die Zusammensetzung wenigstens eine osmotisch und/oder hygroskopisch wirksame Substanz in einer Konzentration von 1 bis 70 Gew.-%. Besonders bevorzugt weist die Zusammensetzung eine osmotisch und/oder hygroskopisch wirksame Substanz in einer Konzentration von 1 bis 20 Gew.%, ganz besonders bevorzugt von 1,5 bis 2,5 Gew.-% auf.

Beispielweise enthält die Zusammensetzung 1 bis 30 % Salz oder 1 bis 70 % Zucker als osmotisch und/oder hygroskopisch wirksame Substanz.

Insbesondere wurde bei Mineralsalz eine sehr gute Wirksamkeit bei mindestens 2 Gew.% Mineralsalz festgestellt.

Durch den Einsatz der genannten Gewichtsanteile an osmotisch und/oder hygroskopisch wirksamer Substanz, insbesondere im Bereich von 1,5 bis 30 Gew.-% in Form eines Salzes, kann eine ausreichende Wirkung auf die Hautreizung erzielt werden, ohne dass die osmotisch wirksame Substanz negative Eigenschaften entfaltet, beispielweise ein Auskristallisieren, ein Austrocknen der Haut oder ein klebriges Gefühl auf der Haut.

Vorzugsweise liegt die osmotisch und/oder hygroskopische Substanz in der Zusammensetzung gelöst oder dispergiert vor. Hierdurch kann eine ausreichende Wirksamkeit der Substanz bei topischer Applikation auf die Haut erzielt werden.

Vorzugsweise enthält die Zusammensetzung zumindest ein Lösungsmittel, insbesondere Wasser als Lösungsmittel. Alternativ oder ergänzend enthält die Zusammensetzung Ethanol als Lösungsmittel, welches zugleich desinfiziert wirkt. Weitere Beispiele für Lösungsmittel sind andere ein- oder mehrwertige Alkohole sowie Polyole, insbesondere Propylenglykol, Pentylenglykol, Butylenglykol. Die Zusammensetzung enthält vorzugsweise ein oder mehrere verschiedene Lösungsmittel. Hierdurch können verschiedene Polaritäten und somit Lösungseffekte ausgenutzt und insbesondere an die osmotisch und/oder hygroskopisch wirksame Substanz angepasst werden.

Zudem kann das Lösungsmittel oder mehrere verschiedene Lösungsmittel derart ausgewählt werden, um lipophile Fremdstoffe und/oder Giftstoffe besser aus der Haut herausziehen zu können. Hierfür kann die Polarität der Zusammensetzung an das Anwendungsgebiet bzw. an den Fremdstoff und/oder das Gift angepasst werden.

In einer bevorzugten Ausführungsform ist die Zusammensetzung als Gel ausgebildet. Hierfür enthält die Zusammensetzung zumindest einen Gelbildner. Diese Form der Formulierung ermöglicht den Einsatz der osmotisch und/oder hygroskopische Substanz in dispergierter Form.

Gele haben die Eigenschaft, gleichzeitig zu einer Wirkung eines enthaltenen Wirkstoffs, einen kühlenden Effekt zu haben.

Zusätzlich stellt Gel eine bevorzugte Applikationsform dar, um eine Anwendung der Zusammensetzung auf der Haut im Bereich der Hautreizung zu gewährleisten, insbesondere im Bereich der Hautreizung.

Vorzugsweise enthält die Zusammensetzung ein Carbomer und/oder ein Cellulosederivat als Gelbildner und Verdicker, insbesondere Hydroxycellulose. Hydroxycellulose ist als halbsynthetischer Gelbildner beispielweise unter der Handelsbezeichnung Natrosol ^{™} 250 HX erhältlich ist.

In alternativen Ausführungsformen kann die Zusammensetzung als Creme, Emulsion oder als Präparat für einen Stick, Roll-on, Pflaster oder ein Spray ausgebildet sein. Diese verschiedenen Applikationsformen ermöglichen den Auftrag oder ein Aufsprühen der Zusammensetzung auf die Haut und gewährleisten einen ausreichend langen Verbleibt der Zusammensetzung auf der Haut, damit ein Fremdstoff, beispielweise ein Sekret oder Nesselgift, aus der Haut gezogen werden kann. Dies basiert auf der Sogwirkung der osmotisch und/oder hygroskopisch wirksamen Substanz insbesondere hervorgerufen durch ein Konzentrationsgefälle.

In einer bevorzugten Ausführungsform weist die Zusammensetzung zumindest ein auf die Hautreizung wirkendes Additiv auf. Vorzugsweise ist das zumindest eine Additiv ausgewählt aus der Gruppe der Feuchthaltemittel, pflanzlichen Extrakte oder Puffer. Hierdurch können weitere vorteilhafte Wirkungen auf die Hautreizung und die umgebende Haut erzielt werden.

Das Feuchthaltemittel ist beispielweise Glycerin und/oder Ethylhexylglycerin. Pflanzliche Extrakte können beispielweise Aloe Vera-Extrakt, Salbeiextrakt oder Spitzwegerichextrakt sein. Als Puffer und zur pH-Regulation kann beispielweise Milchsäure zugesetzt werden.

Die Zusammensetzung kann zudem weitere Wirkstoffe enthalten, Extrakte mit unterstützenden Eigenschaften gegen Rötungen, Entzündungen und/oder mit Antibakterieller Wirkung oder auch Substanzen nicht-pflanzlichen Ursprungs mit den genannten Eigenschaften, beispielweise Cortison. Ein Wirkstoff mit kühlender Eigenschaft ist zudem beispielweise Betain.

Ein kühlender Effekt wird vorzugsweise durch den Einsatz von Aloe Vera Extrakten, Glycerin und/oder Ethanol erhalten. Zudem spenden Aloe Vera-Extrakte und Glycerin der gereizten Haut Feuchtigkeit und pflegen somit dieselbe. Diese Inhaltsstoffe können auch in einer anderen Applikationsform eingesetzt werden, wodurch ein kühlender Effekt unabhängig von Gel-Eigenschaften der Zusammensetzung erzielt wird.

In einer bevorzugten Ausführungsform enthält die Zusammensetzung zumindest folgende Inhaltsstoffe:
80 - 99 Gew.-% Lösungsmittel und 1-20 Gew. %, bevorzugt 1,5 bis 2,5 Gew. %, besonders bevorzugt 2-Gew.-% osmotisch und/oder hygroskopisch wirksame Substanz.

Das Lösungsmittel kann dabei beispielweise durch 0,1 - 60 Gew.-% Ethanol und die Restmenge an Wasser gebildet sein.

In einer bevorzugten Weiterbildung enthält die gelförmige Zusammensetzung zumindest folgende Inhaltsstoffe:
80 - 99 Gew.-% Lösungsmittel, 1 - 20 Gew. %, bevorzugt 1,5 bis 2,5 Gew. %, besonders bevorzugt 2-Gew.-% osmotisch und/oder hygroskopisch wirksame Substanz und 0,1 - 5 Gew.-%, bevorzugt 1 - 2 Gew.-%, besonders bevorzugt 1,5 Gew.-% Gelbildner.

In einer bevorzugten Weiterbildung enthält die gelförmige Zusammensetzung mit Additiven zumindest folgende Inhaltsstoffe:
80 - 99 Gew.-% Lösungsmittel, 1 - 20 Gew. %, bevorzugt 1,5 bis 2,5 Gew. %, besonders bevorzugt 2-Gew.-% osmotisch und/oder hygroskopisch wirksame Substanz und 0,1 - 5 Gew.-%, bevorzugt 1 - 2 Gew.-%, besonders bevorzugt 1,5 Gew.-% Gelbildner, 0,0001 - 20 Gew.-% Additive.

In einer weiteren bevorzugten Weiterbildung enthält die gelförmige Zusammensetzung mit Additiven zumindest folgende Inhaltsstoffe:
- 80 - 99 Gew.-% Lösungsmittel,
- 1-20 Gew. %, bevorzugt 1,5 bis 2,5 Gew. %, besonders bevorzugt 2-Gew.-% osmotisch und/oder hygroskopisch wirksame Substanz,
- 0,1 - 5 Gew.-%, bevorzugt 1 - 2 Gew.-%, besonders bevorzugt 1,5 Gew.-% Gelbildner und
- 0,0001 - 20 Gew.-% Additive, insbesondere:
   ∘ Feuchthaltemittel 0,1 - 20 Gew.-%, bevorzugt 1 - 5 Gew.-%, besonders bevorzugt 3 Gew.-%,
   ∘ Pflanzlichen Extrakten 0,0001 - 20 Gew.-%, bevorzugt 0,01 Gew.-% bis 1,5 Gew.-% und
   ∘ Puffer nach Bedarf, abhängig vom pH-Wert der Zusammensetzung 0,1 - 5 Gew.-%.

In einer bevorzugten Ausführungsform besteht die Zusammensetzung aus folgende Inhaltsstoffe:
- 80 - 99 Gew.-% Lösungsmittel, insbesondere:
   ∘ Ethanol 0,1 - 60 Gew.-%, besonders bevorzugt 20 %
   ∘ Wasser 20 - 99 Gew.-%, insbesondere zum Auffüllen auf 100 %
- 1-20 Gew. %, bevorzugt 1,5 bis 2,5 Gew. %, besonders bevorzugt 2-Gew.-% osmotisch und/oder hygroskopisch wirksame Substanz, insbesondere Mineralsalz
- 0,1 - 5 Gew.-%, bevorzugt 1 - 2 Gew.-%, besonders bevorzugt 1,5 Gew.-% Gelbildner, insbesondere Hydroxycellulose und
- 0,0001 - 20 Gew.-% Additive, insbesondere:
   ∘ Feuchthaltemittel 0,1 - 20 Gew.-%, bevorzugt 1 - 5 Gew.-%, besonders bevorzugt 3 Gew.-%, insbesondere Glycerin als Feuchthaltemittel
   ∘ Pflanzlichen Extrakten 0,0001 - 20 Gew.-%, bevorzugt 0,01 Gew.-% bis 1,5 Gew.-%, insbesondere:
      ▪ Spitzwegerichextrakt zu 0,1 - 20 Gew.-%, besonders bevorzugt 1 Gew.-% und
      ▪ Aloe Vera-Extrakt zu 0,001 - 5 Gew.-%, besonders bevorzugt zu 0,03 Gew.-%
   ∘ Puffer nach Bedarf, insbesondere Milchsäure, abhängig vom pH-Wert der Zusammensetzung 0,1 - 5 Gew.-%.

Zudem betrifft die Erfindung die Verwendung einer Zusammensetzung mit zumindest einer osmotisch wirksamen Substanz zur Behandlung von Hautreizungen, welche durch pflanzliche oder tierische Einwirkungen auf oder in der Haut verursacht werden.

Vorzugsweise ist die Zusammensetzung direkt nach Auftreten eines Insektenstichs, Spinnenbisses oder Kontakt mit Nesselgift anzuwenden. Unerwarteterweise wirkt die Zusammensetzung auch bei nicht sofortigem Auftragen, sondern auch noch mehreren Stunden und nachdem bereits eine starke Schwellung aufgetreten ist.

In einer bevorzugten Ausführungsform wird die Zusammensetzung als Gel appliziert. Hierdurch kann der Wirkstoff in Form der osmotisch und/oder hygroskopisch wirksamen Substanz gezielt und anhaltend appliziert werden.

Weitere Merkmale, Einzelheiten und Vorteile der Erfindung ergeben sich aus dem Wortlaut der Ansprüche sowie aus der folgenden Beschreibung von Ausführungsbeispielen und Figuren.

Es zeigen:

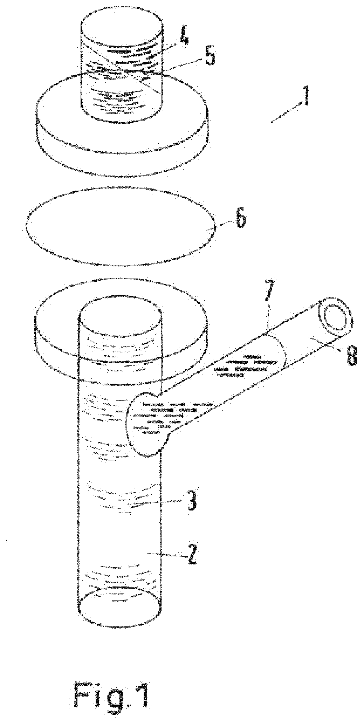

- Fig. 1: Explosionszeichnung einer Franz-Diffusionskammer

### Untersuchungen der Wirkstoffeigenschaften -

Nachweis der osmotischen Aktivität mittels der Franz-Diffusions-Kammer

Ziel des Versuches war es zu demonstrieren, dass die Zusammensetzung geeignet ist, mittels osmotisch wirksamer Substanz Fremdstoffe, welche Hautreizungen verursachen, aus der Haut herauszuziehen.

Hierfür wurde eine beispielhafte Zusammensetzung in Form eines Gels mit 2 Gew.% Salz als osmotisch wirksame Substanz, gelöst in Wasser als Lösungsmittel und versetzt mit Hydroxycellulose als Gelbildner, gegenüber einer physiologischen Kochsalzlösung (0,9%ige Kochsalzlösung) getestet. Der Austausch erfolgt über eine semipermeable Membran mit winzigem Einstichloch zur Simulation der Haut mit einer Einstich- oder Bissstelle. Als Salz wurde Mineralsalz einer Thermalquelle der Stadt Bad Ems, insbesondere Emser^{®} Salz, benutzt.

Die Wirkung der Zusammensetzung, ein Entfernen von Fremdstoffen, in Form von Sekreten oder Giften, aus der Haut mittels osmotisch wirksamer Substanz und Konzentrationsgradient, wurde mittels einer Franz-Diffusionskammer 1 gezeigt. Eine Explosionszeichnung einer derartige Franz-Diffusionskammer 1 ist in Fig. 1 gezeigt. Als Referenzzusammensetzung 2 wurde zur Modellierung des Blutes eine 0,9% Kochsalzlösung in die Akzeptorkammer 3 gegeben, welche zur besseren Sichtbarkeit mit dem membrangängigen Farbstoff Fluoreszein-Natrium (C.I.45350) angefärbt wurde.

Die Donorkammer 4, in welche die Testzusammensetzung 5 gegeben wurde, war mit der Akzeptorkammer 3 über eine Membran 6 verbunden. In die Membran wurde zur Simulation des Stichkanals ein winziges Loch mit einer Kanüle gestochen.

Als erfindungsgemäße Zusammensetzung wurde eine gelförmige Zusammensetzung mit 2 Gew.% Salz als osmotisch und hygroskopisch wirksame Substanz, gelöst in Wasser als Lösungsmittel und Hydroxyethylcellulose als Gelbildner als Testzusammensetzung 5 benutzt.

Als Kontrolle wurde in einer separaten Franz-Diffusionskammer 1 die Akzeptorkammer 3 ebenfalls mit 0,9% Kochsalzlösung und membrangängigen Farbstoff und eine nichterfindungsgemäße Zusammensetzung ohne osmotisch oder hygroskopisch wirksame Substanz und Gelbildner als Testzusammensetzung 5 in die Donorkammer 4 gefüllt.

Über einen Zeitraum von 120 Minuten wurde in regelmäßigen Abständen ein Foto der jeweiligen gefüllten Franz-Diffusionszellen 1 gemacht, um den Osmoseprozess zu dokumentieren.

Im Falle der erfindungsgemäßen Zusammensetzungen mit osmotisch wirksamer Substanz, sank der anfängliche Flüssigkeitspegel 7 in der Akzeptorkammer 3 aufgrund von Osmosegetriebener Diffusion durch die Membran 6. Dies lässt sich an einem Abfallen des anfänglichen Flüssigkeitspegels 7 im Indikator-Röhrchen 8 ablesen.

Ohne osmotisch wirksame Substanz in der nicht-erfindungsgemäßen Zusammensetzung als Kontrolle gelangt Flüssigkeit aus der oberen Donorkammer 4 in die untere Akzeptorkammer 3 und der Flüssigkeitspegel steigt.

Dies zeigt deutlich, dass die getestete erfindungsgemäße Zusammensetzung mit osmotisch und hygroskopisch aktiver Substanz aufgrund eines Konzentrationsgefälles in der Lage ist, Flüssigkeit aus einer physiologischen Kochsalzlösung durch einen simulierten Stichkanal zu ziehen.

Umgekehrt führt eine Substanz ohne osmotisch wirksame Substanz dazu, dass eine umgekehrte Diffusion von Flüssigkeit hin zu der physiologischen Salzlösung zu beobachten ist.

Die Erfindung ist nicht auf eine der vorbeschriebenen Ausführungsformen beschränkt, sondern in vielfältiger Weise abwandelbar. Insbesondere sind die im Zusammenhang mit der Zusammensetzung genannten Merkmale und Vorteile auf die Verwendung einer entsprechenden Zusammensetzung übertragbar und umgekehrt.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Zusammensetzung zur Anwendung bei der Behandlung von Hautreizungen, welche durch pflanzliche oder tierische Einwirkungen auf oder in der Haut verursacht werden, wobei die Zusammensetzung wenigstens eine osmotisch und/oder hygroskopisch wirksame Substanz enthält.

2. Zusammensetzung nach Anspruch 1, **dadurch gekennzeichnet, dass** die wenigstens eine osmotisch und/oder hygroskopisch wirksame Substanz, ein Salz oder ein Zucker ist.

3. Zusammensetzung nach einem der vorhergehenden Ansprüche, **dadurch gekennzeichnet, dass** die Zusammensetzung die wenigstens eine osmotisch und/oder hygroskopisch wirksame Substanz in einer Konzentration von 1 bis 30 Gew.-%, bevorzugt in einer Konzentration von 1,5 bis 2,5 Gew.-% enthalten sind.

4. Zusammensetzung nach einem der vorhergehenden Ansprüche, **dadurch gekennzeichnet, dass** die Zusammensetzung zumindest ein Lösungsmittel enthält, insbesondere Wasser als Lösungsmittel.

5. Zusammensetzung nach einem der vorhergehenden Ansprüche, **dadurch gekennzeichnet, dass** die Zusammensetzung zumindest einen Gelbildner enthält.

6. Zusammensetzung nach einem der vorhergehenden Ansprüche, **dadurch gekennzeichnet, dass** zumindest ein Additiv enthalten ist.

7. Zusammensetzung nach Anspruch 5, **dadurch gekennzeichnet, dass** die Zusammensetzung zumindest ein Additiv ausgewählt aus der Gruppe: Feuchthaltemittel, pflanzliches Extrakt und Puffer.

8. Verwendung einer Zusammensetzung mit zumindest einer osmotisch wirksamen Substanz zur Behandlung von Hautreizungen, welche durch pflanzliche oder tierische Einwirkungen auf oder in der Haut verursacht werden.

9. Verwendung einer Zusammensetzung nach Anspruch 8, **dadurch gekennzeichnet, dass** die Zusammensetzung als Gel appliziert wird.
